(19) Europäisches Patentamt

European Patent Office

Office européen des brevets

(11) **EP 3 949 850 A1**

(12) **EUROPEAN PATENT APPLICATION**

(43) Date of publication:
**09.02.2022 Bulletin 2022/06**

(21) Application number: **20189718.8**

(22) Date of filing: **05.08.2020**

(51) International Patent Classification (IPC):
**A61B 5/0432** *(2006.01)* **A61B 5/0404** *(2006.01)*
**A61B 5/0428** *(2006.01)* **A61B 5/0408** *(2006.01)*
**A61B 5/00** *(2006.01)*

(52) Cooperative Patent Classification (CPC):
**A61B 5/7225; A61B 5/282; A61B 5/333;**
A61B 5/30; A61B 5/332

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **MedBeat Sweden AB
211 19 Malmö (SE)**

(72) Inventor: **Rask, Erik Karl Peter
222 40 Lund (SE)**

(74) Representative: **Høiberg P/S
Adelgade 12
1304 Copenhagen K (DK)**

(54) **MOBILE ELECTROCARDIOGRAPHY RECORDING DEVICE**

(57) The present disclosure relates to a portable electrocardiography device comprising: a housing; a battery; a first memory; an arrangement of electrodes located on a first plane defined by a first side of the housing, including: three ECG electrodes arranged in a triangular pattern; a common mode rejection electrode located in the center of the triangular pattern; and a processing unit in electrical communication with the electrodes, said processing unit configured for receiving an ECG signal from each electrode and processing the ECG signals into ECG data.

**Description**

The present disclosure relates to portable electrocardiography measurement devices that may be able to perform electrocardiographic recordings for an extended amount of time, and methods for use thereof.

**Background of invention**

Electrocardiography is the process of producing an electrocardiogram. It is a graph of voltage versus time of the electrical activity of the heart using electrodes placed on the skin. These electrodes detect the small electrical changes that are a consequence of cardiac muscle depolarization followed by repolarization during each cardiac cycle. Changes in the normal ECG pattern occur in numerous cardiac abnormalities, including cardiac rhythm disturbances, inadequate coronary artery blood flow, and electrolyte disturbances.

A standard electrocardiography device typically records the heart's electrical activity for only a few seconds. This can detect abnormalities that are constant; however, sometimes abnormal heart rhythms and inadequate blood flow to the heart muscle occur only briefly or unpredictably. These types of problems required prolonged measurements of the heart's electrical activity. One type of device that may be used for this purpose is continuous ambulatory electrocardiography, in which the ECG is recorded continuously for typically 24 to 48 hours while the person engages in normal daily activities. Another approach for capturing rare events is the use of event-based electrocardiography, in which the ECG is continuously monitored for an extended amount of time, for example up to a month, but the data is only stored when the device receives a trigger from the user that an abnormal event has occurred.

While these types of device have provided means for acquiring rare events, they are typically bulky and offer limited resolution, and thereby the measurement data may not provide sufficient details of the heart's electrical activity in order to gain a clinician insights into a user's suspected condition. There is consequently a significant need for a small and portable device that is able to acquire electrocardiography data for a prolonged time, with sufficient detail in order to correctly diagnose a user's suspected condition.

**Summary of invention**

The present inventor has realized that electrocardiography devices can be made significantly more portable, and easier to use, while still being capable of providing high resolution measurement data.

The present invention therefore in a first aspect relates to a portable electrocardiography device comprising:

- a housing;
- a battery;
- an arrangement of electrodes located on a first plane defined by a first side of the electrocardiography device, including:

  - three ECG arranged in a triangular pattern;
  - a common mode rejection electrode located in the center of the triangular pattern; and

a processing unit in electrical communication with the electrodes, said processing unit configured for receiving an ECG signal from each electrode and processing the ECG signals into ECG data.

The three ECG electrodes may be provided at fixed locations of the housing, preferably at each corner of a triangular housing. With the ECG electrodes provided at fixed locations with respect to the housing, the distances and angles between the ECG electrodes may have already been set as part of the construction of the electrocardiography device. The distances and angles between the ECG electrodes may thereby not be affected by how the ECG electrodes are attached to the user. This is in stark contrast to conventional electrocardiography devices that typically rely on the use of electrical cables from a remote located device to the ECG electrodes.

The ECG electrodes may thereby be provided in a suitable pattern for obtaining high resolution ECG signals. For example, the electrocardiography device may be provided with three ECG electrodes that form an equilateral triangular pattern. Said pattern may comprise sides that are less than 15 cm in length. The device may thereby be small and portable, offering the user an ability of recording ECG signals for an extended period of time.

The ECG device preferably comprises a Wilson reference generator circuit that is used in a driven right leg configuration. The output of the Wilson reference generator circuit is expected to be the common mode interference. By inversion of this signal and provided to the common mode rejection electrode, the common mode interference is actively suppressed.

The ECG electrodes preferably comprises a first plane defined by a first side of the housing, and wherein the ECG electrodes are located at each corner of said first side of the housing. The electrocardiography device may further be configured to have ECG electrodes that are to be located at positions of a chest that have a low degree of muscles, fat tissue and breast tissue.

The electrocardiography device may for example be configured to comprise two ECG electrodes adapted to be attached symmetrically across the sternum of a user, and may comprise a third ECG electrode that is adapted to be attached to an inferior centrally located position of the sternum. Such a configuration and positioning of said device, may lead to an avoidance of the parts of the chest of the user where there is an abundance of muscle tissue, fat tissue and/or breast tissue. Signals obtained from the two symmetrically superior located electrodes may be used to suppress noise from electrical pulses to the muscles but additionally also artefacts due to movements of the user.

The location of the ECG electrodes allows for a larger variation between the shape of users and their hearts, while maintaining high signal-to-noise measurements, as compared to conventional electrocardiography devices. The portable electrocardiography device may thereby result in more accurate P-wave, QRS-complex and ST segments than these conventional electrocardiography devices.

The electrocardiography device preferably comprises means for wireless communication, for example with a cloud-based server and/or a personal computing device, such as a smartphone or a tablet. The electrocardiography device may be configured such that the following attachment to the chest area of the user, said user may communicate with the electrocardiography device through the personal computing device. The user may for example request that ECG data is provided from the electrocardiography device to the personal computing device, for displaying on said personal computing device and thereby ensuring correct electrode placement and operation.

The electrocardiography device may further be configured to receive a first user input from the user, for registration of an occurrence of an abnormal event. For example chest pain or heart palpitation. The electrocardiography device is preferably configured such that it, upon receiving said first user input, stores a recording related to said registration. The stored data may for example be in the form of a time point, or the stored data may be in the form of ECG data provided from ECG signals retrieved in a time interval defined by a predetermined time period before receiving the first user input and a predefined time interval after receiving said first user input.

The electrocardiography device may further be equipped with means for communication with a remote server, such as over an LTE Cat M1 network. By communication with a remote server, the electrocardiography device may be able to transmit registered and/or processed data, such as ECG data, to said remote server. The ECG data uploaded to the cloud server may be further processed, for example into multiple additional leads, and investigated by physicians through a web application.

In a second aspect, the present invention relates to a method for electrocardiographic measurements of a user, comprising:

- providing a portable electrocardiography device comprising an arrangement of electrodes located on a first plane defined by a first side of the housing, including:

    ∘ three ECG electrodes arranged in a triangular pattern; and
    ∘ a common mode rejection electrode located in the center of the triangular pattern;

- positioning said portable electrocardiography device on the chest of the user; and
- acquiring ECG signals from said electrodes;

thereby performing electrocardiographic measurements of the user.

It is a preference that the electrocardiography device is configured such that the ECG electrode may be placed on the user in an equilateral triangular pattern, wherein each side of said triangular pattern is no more than 15 centimeters.

It is a further preference that the electrocardiography device is positioned on said user symmetrically on the user, such as in order to avoid areas with fat tissue, muscle tissue and/or breast tissue.

**Description of drawings**

**Fig. 1** shows an infrastructure for accurate ECG measurement by a portable electrocardiography device.
**Fig. 2** shows a number of user interfaces of a smartphone app for presenting ECG data, obtaining status of the ECG device, and registering of events.
**Fig. 3** shows a number of user interfaces of a web application for use by a medical professional comprising ECG data obtained during an extended amount of time.
**Fig. 4** shows a schematic illustration of the ECG device, comprising an ECG pad and a housing.
**Fig. 5** shows a block diagram describing the process for continuously obtaining a common mode interference signal.

**Detailed description of the invention**

The present invention relates, in a first aspect, to an electrocardiography device that can be used for an extended period of time, and thereby register and/or detect the electrical activity of the heart for an extended period of time. Said electrocardiography (ECG) device is preferably configured such that it can be worn with ease. For example, the electrocardiography device may not be heavier than that it can be attached to the chest with suitable means, such as by adhesive, without discomfort to the user. The ECG may further be shaped in a way that it can easily be worn under garments, such as clothing, i.e. said electrocardiography device is not bulky. It is a further preference that the electrocardiography device consists of a single unit, without electrical wiring to secondary battery units and/or recording units. Instead it is a preference that the electrocardiography device is configured as a compact and light-weight portable device, that a user may wear and/or carry around with ease for an extended amount of time.

In an embodiment of the present disclosure, the electrocardiography device comprises a housing. It is a preference that the housing is substantially planar, having a first side, for facing the user when in use, and a second side for facing away from the user when in use. The housing of the electrocardiography device may comprise visual indicators of the present status of the electrocardiography device . For example if the battery needs to be replaced within a certain amount of time and/or if any of the memories of the electrocardiography device , for storing data, is about to be full. The housing of the electrocardiography device may further be substantially waterproof, such as waterproof. This may be achieved by the use of a housing that is adapted such that only electrical contacts are exposed to the surrounding environment of the housing. For example, the housing may comprise a number of apertures, at which said electrical contacts may be positioned. The electrical contacts and/or the housing may be sealed, such that the electrocardiography device is capable of withstanding water for an extended amount of time. As previously mentioned, the electrocardiography device may be configured such that it allows for a user to wear and use the electrocardiography device without interfering with the normal daily routines of the user. Thereby, by making the electrocardiography device small, lightweight, and waterproof the user may be able to carry out the normal daily routines, such as showering, without having to take any special considerations to the electrocardiography device .

In a further embodiment of the present disclosure, the electrocardiography device comprises a battery. Preferably said battery is rechargeable, by for example wired and/or wireless charging. The battery may for example be adapted for wireless charging by inductive coupling, resonant inductive coupling and/or capacitive coupling.

In a further embodiment of the present disclosure, the electrocardiography device comprises a first memory, such as for storing ECG data and/or ECG signals. It is a preference that the capacity of the first memory is selected such that the electrocardiography device is capable of storing continuous ECG data obtained during at least 7 days, more preferably at least 15 days, yet more preferably at least 30 days.

In a preferred embodiment of the present disclosure, the electrocardiography device comprises an arrangement of electrodes located on a first plane defined by a first side of the housing. Said arrangement of electrodes may consist or comprise of a number of electrodes such as three or four. In a preferred embodiment of the present disclosure, the arrangement of electrodes consists of three ECG electrodes and a Common mode rejection electrode. It is a further preference that the ECG electrodes are located on the first side of the housing of the electrocardiography device . It is a further preference that the three ECG electrodes are located in a triangular pattern. In yet a preferred embodiment of the present disclosure, the electrocardiography device comprises a Common mode rejection electrode that is located in the same plane as the ECG electrodes. In a further preferred embodiment of the present disclosure the Common mode rejection electrode is located substantially in the center of the triangular pattern formed by the ECG electrodes. While the ECG electrodes may form a triangular pattern, it should be noted that the housing may not necessarily be provided in a similar shape. Instead it may be a preference that the housing is provided in a shape that minimizes the area of the first side, and/or minimizes the projected area of the electrocardiography device onto a surface parallel with the first surface. Thereby, the device may be provided in for example a substantially three bladed shape, such as three bladed boomerang and/or a three bladed propeller. The housing of the electrocardiography device may thereby have three arms extending outwards from the centrally located Common mode rejection electrode. Each arm of said three arms may have any length independent of the other arms, and may further extend outward from the central part of the housing at any angle. The special case being wherein each arm forms a 60 degree angle to the adjacent arm, and wherein the length of each arm is identical. In this configuration the pattern of the electrodes may form an equilateral triangle.

In a further embodiment of the present disclosure, the electrocardiography device comprises a processing unit. Said processing unit may be in electrical communication, such as direct electrical communication, with the electrodes, such as each electrode. It is a preference that the processing unit is configured such that it is able to receive ECG signals from each electrode. It is a further preference that the processing unit is configured for processing of the ECG signals, such as into ECG data.

aid processing unit configured for receiving an ECG signal from each electrode and processing the ECG signals into ECG data.

*Communication means*

In a further embodiment of the present disclosure, the electrocardiography device comprises means for wireless communication, such as means for communication over a mobile network, such as 4G LTE and/or 5G. Furthermore, said means for wireless communication may comprise means for communication by Bluetooth, such as Bluetooth Low Energy. Bluetooth Low Energy (BLE) uses the same 2.4 GHz radio frequencies as classic Bluetooth, which allows dual-mode devices to share a single radio antenna, but uses a simpler modulation system. It is a preference that the electrocardiography device comprises at least one means for long-range communication, such as LTE, and one means for short-range communication, such as BLE. Thereby, the electrocardiography device may be able to communicate with electronic devices that are in proximity of the electrocardiography device and/or user, and furthermore communicate with remote devices, such as remote servers. The electrocardiography device may for example be able to communicate with a personal computing device, such as a smartphone, that is in proximity of the electrocardiography device , for example by BLE. Furthermore, the electrocardiography device may be able to communicate with a remote server, such as a cloud server, over LTE. Personal computing device may be any device that may be operated by an end-user, and may include smartphones, tablets, stationary computers, such as a desktop computer, portable computers, such as a laptop. In a preferred embodiment of the present disclosure, the personal computing device is provided with software for displaying an interface to the user for allowing communication with the electrocardiography device , such as retrieval of the electrocardiography device status, present ECG data, as well as past ECG data recordings.

In a further embodiment of the present disclosure, the electrocardiography device is configured such that it may communicate with remote devices through devices in its proximity. Thereby, for example, the electrocardiography device may communicate by Bluetooth with a smartphone that, in turn, communicates over mobile networks with a remote server.

*First user input*

In a further embodiment of the present disclosure, the electrocardiography device is configured for receiving a first user input. Said first user input may be provided to the electrocardiography device by direct interaction with the electrocardiography device , such as by the push of a button extending out from an aperture in the housing of the electrocardiography device , by voice command, and/or touch sensitive buttons/or screens on a surface of the housing. Furthermore, a user may interact indirectly with the electrocardiography device , such as by use of the personal computation device. The user may thereby interact with the personal computation device which, in turn, send commands to the electrocardiography device . The first user input may thereby be provided by the user through the interaction with the personal computation device and transmitted to the electrocardiography device by the personal computation device.

In a further embodiment of the present disclosure, the electrocardiography device is configured to, upon receiving the first user input, store ECG data and/or ECG signals. The user may, prior to use, have been instructed to, upon notice of an abnormality, such as palpitation and/or chest pain, provide the electrocardiography device with the first user input, such as by interacting with the personal computation device. The electrocardiography device may be configured to, upon receiving the first user input, store ECG data and/or ECG signals obtained during a recording interval, said recording interval may be defined by a predetermined time before receiving said first user input and a predetermined time after receiving said first user input. The recording interval may be symmetric around the time point of providing the first user input, such as 5 minutes before said provision and 5 minutes after said provision. The recording interval may further be asymmetrically disposed in time around the time point of providing the first user input, such as 7 minutes before said provision and 2 minutes after said provision.

In a further embodiment of the present disclosure, the electrocardiography device is configured to, upon receiving the first user input, store ECG data and/or ECG signals to a second memory. It should be noted that the first memory and the second memory may be different partitions of the same memory unit, or may be two separate physical memory units. It is a preference that the electrocardiography device comprises both the first and the second memory. During use, the electrocardiography device may be configured to store ECG signals and/or ECG data on the first memory in a loop recording manner. ECG recordings may thereby be carried out continuously and ECG signals and/or ECG data may be continuously stored on the first memory. For loop storage of the ECG data and/or ECG signals, the data quality, such as the sampling rate, and the memory size determines how old recordings are present on the first memory before being written over. In one embodiment of the present disclosure, the first memory has a capacity to store at least data obtained during a time interval equal to the recording interval. In an alternative embodiment of the present disclosure, the present disclosure is capable of recording during a substantial amount of time, such as at least 7 days. If the intended observation period, during which the user is to wear the electrocardiography device , is less than the storage capability of the first memory there may not be a need for a second memory. Therefore, in a further embodiment of the present disclosure, the electrocardiography device is configured to, upon receiving the first user input, to register/add a time point to the ECG data. The time point may thereafter be stored, together with the remainder of the ECG data. The time point may thereafter serve the purpose of indicating when the user noticed the occurrence of an abnormal event, such as palpitations or chest pain.

In a further embodiment of the present disclosure, the device is configured to, upon receiving a second user input, continuously transmit ECG data to the personal computing device, such as low-resolution ECG data for ensuring correct placement of the electrodes.

In a further embodiment of the present disclosure, the said device is configured to periodically, such as upon recharging of the battery, transmit stored ECG data, such as from the secondary memory, to the cloud-based server.

*Electrical components*

In a further embodiment of the present disclosure, the electrocardiography device is configured for continuously receiving ECG signals from the ECG electrodes. In yet a further embodiment of the present disclosure the electrocardiography device is configured for continuously processing ECG signals into ECG data. The electrocardiography device may for example comprise a processing unit that is configured for continuously receiving ECG signals from the ECG electrodes. Furthermore the processing unit may be configured for continuously processing of ECG signals into ECG data.

ECG signals may be the signals received from the electrodes, such as the ECG electrodes and/or the Common mode rejection electrode. Preferably the processing unit is configured to receive and/or process the ECG signals from the ECG electrodes and the Common mode rejection electrode. The processing unit may process the ECG signals into ECG data and preferably comprises at least analog-to-digital conversion. Furthermore, data may be removed and/or added in the processing, for example time points may be added to the ECG data by for example an on-board real time clock.

In a further embodiment of the present disclosure, the electrocardiography device is configured to automatically and continuously receive and/or process ECG signals upon connecting each ECG electrode to an electrode pad. The electrocardiography device may thereby require a minimal amount of interaction from a user, instead the only requirement is that the electrocardiography device is connected to the electrode pads. Preferably at least the ECG electrodes, such as at least the ECG electrode and the Common mode rejection electrode. Thereby, if the electrocardiography device detects that there is no electrical connection to an electrode pad, the recording may not start. It is a preference that the electrode pad is provided in a material that allows for good adhesion to the skin of the user in addition to allowing normal skin respiration. The electrode pad may for example be provided in a soft cloth material that allows the skin to breathe and stretch with the movement of the user. Alternatively the electrode pads may be provided in a non-porous backing material. In a preferred embodiment of the present disclosure the electrodes and/or the electrode pad is provided in Ag/AgCl. In a preferred embodiment the electrode pads are Ag/AgCl electrodes.

In a further embodiment of the present disclosure, the electrocardiography device is configured for loop recording of ECG data, such as on a first memory. The electrocardiography device may thereby, depending on the quality of the ECG data, such as the sampling rate, and the capacity of the first memory, store data during a specific time period before the latest processed data replaces the first processed data. Loop recording may thereby allow for continuous recording even if the first memory reaches full capacity.

In a further embodiment of the present disclosure, the electrocardiography device is configured to receive the first user input from the personal computation device, input means located on said electrocardiography device, such as a button or touch screen, and/or by voice command from a user. The electrocardiography device may thereby comprise a microphone to register voice commands.

It is a preference that the battery has a capacity that allows the electrocardiography device to continuously record and/or measure ECG signals without being recharged and/or replaced. It is a preference that the electrocardiography device is capable of being used while in charging mode. Thereby, the electrocardiography device may continuously retrieve data, even if the electrocardiography device comprises a rechargeable battery that is being recharged. Recharging of the electrocardiography device may be performed by wired and/or wireless charging.

*CMR*

A driven right leg circuit or DRL circuit is an electric circuit that is often added to biological signal amplifiers to reduce common-mode interference. Biological signal amplifiers such as ECG (electrocardiogram) EEG (electroencephalogram) or EMG circuits measure very small electrical signals emitted by the body, often as small as several micro-volts (millionths of a volt). Unfortunately, the user's body can also act as an antenna which picks up electromagnetic interference, especially 50/60 Hz noise from electrical power lines. This interference can obscure the biological signals, making them very hard to measure. Right leg driver circuitry is used to eliminate interference noise by actively cancelling the interference.

The right leg drive (RLD) is a programmable operational amplifier that is intended to control the common-mode level of the user of the ECG device and thereby improving the AC CMRR of the entire system. In a typical application, the common-mode level of the user's body is measured by a Common-Mode Detector. When used in an inverting amplifier topology, the common mode rejection electrode is driven by the RLD to counter any differences between the reference voltage and the detected common-mode level. This reduces the amount of power-line common-mode interference.

In an embodiment of the present disclosure, the ECG device features a Wilson reference block, such as consisting of three buffer amplifiers and resistors, that can generate the voltages for the Wilson Central Terminal and/or Goldberger terminals.

In a further embodiment of the present disclosure the Common-mode detector circuit is a Wilson reference generator circuit.

For the Wilson Central Terminal, there are three main ECG leads that are measured differentially, Lead I (LA-RA), Lead II (LL-RA), Lead III (LL-LA). The Wilson Central Terminal is defined as the average of the three limb electrodes, RA, LA and LL, thereby the Wilson Central Terminal is (RA+LA+LL)/3. This is typically a preferred output of the Wilson Reference Generator Circuit, which is the sum of ($BUF_1$ + $BUF_2$ + $BUF_3$)/3. Each of the buffer amplifiers, $BUF_1$, $BUF_2$, and $BUF_3$ may be connected to one of the ECR electrodes, thereby $BUF_1$ may be connected to RA, $BUF_2$, may be connected to LA, and $BUF_3$, connected to LL. A non-zero Wilson Central Terminal is expected to have been caused by common-mode interference. Thereby, an inverted common-mode interference signal applied to the user may advantageously be used to suppress the common-mode interference.

The right leg drive (RLD) may be a programmable operational amplifier that is intended to control the common-mode level of the user of the ECG device and thereby improving the AC CMRR of the entire system. In a typical application, the common-mode level of the user's body is measured by a Common-Mode Detector. When used in an inverting amplifier topology, the common mode rejection electrode may be driven by the RLD to counter any differences between the reference voltage and the detected common-mode level. This reduces the amount of power-line common-mode interference.

The negative input terminal of the RLD op-amp may always be connected to the RLDINV pin. By default, the positive input terminal of the RLD op-amp may be routed to the RLDIN pin. However, the positive input terminal may be routed internally to the RLD reference. This will allow connecting the output of the analogue pace instrumentation amplifier to the RLDIN pin. The output of the RLD operational amplifier may always be connected to the RLDOUT pin, and in addition, can be connected to one of the IN1-IN6 terminals.

In an embodiment of the present disclosure, the common mode rejection electrode may be driven by the RLD to counter any differences between the reference voltage and the detected common-mode level. This may thereby act to reduce the amount of power-line common-mode interference. Typically, the ECG device comprises three measurement electrodes providing three in signals, such as IN1, IN2, and IN3, each being the ECG signal of each electrode. The in-channels (IN1, IN2, and IN3) are used in a driven right leg circuit in order to eliminate measurement noise by actively cancelling the common-mode interference. These in signals are thereby used to derive an inverted signal of the common-mode interference. The in signals may be provided to a Wilson reference generator circuit. First, said inputs may be routed to a Wilson reference electrode amplifier that filters out the background noise. This noise may for example be the sum of Lead I, Lead III, -Lead II. Given the conventional notation, a non-zero sum of the above equation represents noise. By routing the output of the Wilson reference electrode amplifier (Wilson ref.) through the Wilson reference output (WCT) and the Right-leg drive amplifier negative to the RLD amplifier. The measured noise may be used to produce an inverted signal of the noise, that is output to the Right-leg drive amplifier output (RLDOUT). This signal, the inverted noise signal, may thereafter be fed to the common mode rejection electrode for active cancelling of the common-mode interference.

For the Wilson Central Terminal, there are three main ECG leads that are measured differentially, Lead I (LA-RA), Lead II (LL-RA), Lead III (LL-LA). The Wilson Central Terminal is typically defined as the average of the three limb electrodes, RA, LA and LL, thereby the Wilson Central Terminal may be defined as (RA+LA+LL)/3. This may be the output of the Wilson Reference Generator Circuit, which is the sum of ($BUF_1$ + $BUF_2$ + $BUF_3$)/3. Each of the $BUF_1$, $BUF_2$, and $BUF_3$ are connected to one of the ECR electrodes, thereby $BUF_1$ may be connected to RA, $BUF_2$, may be connected to LA, and $BUF_3$, connected to LL. In an embodiment of the present disclosure, the Wilson Central Terminal is inverted and applied to the common mode rejection electrode.

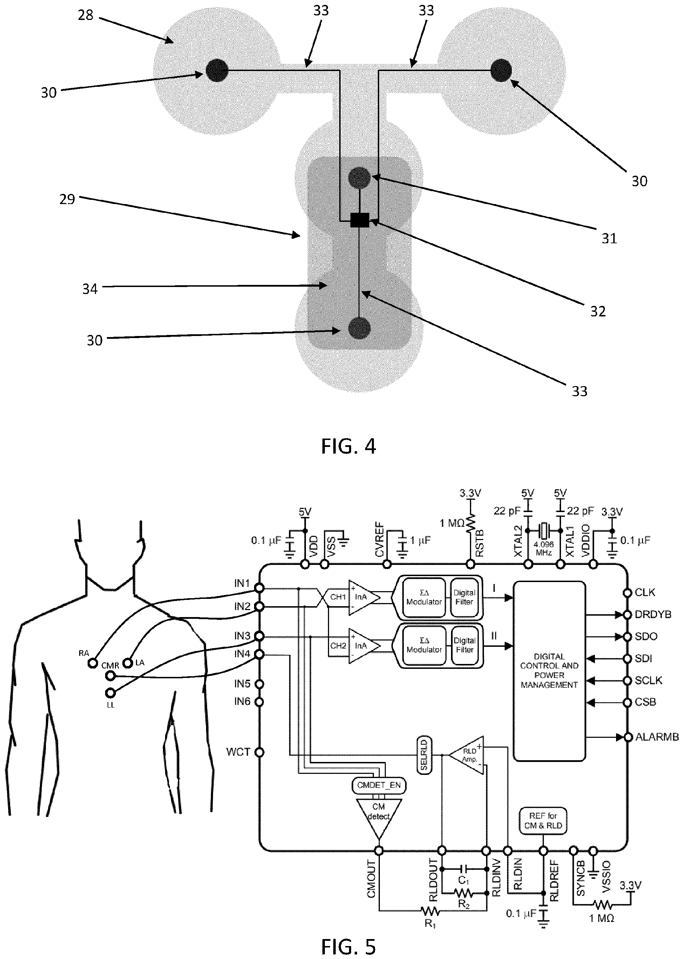

In an embodiment of the present disclosure, the right-arm (RA), left-arm (LA), left-leg (LL) and right-leg (RL) electrodes are connected to the IN1, IN2, IN3 and IN4 pins respectively, as shown in Fig. 5. The ECG device may be configured to use the Common-Mode Detector to measure the common-mode of the system by averaging the voltage of input pins IN1, IN2 and IN3, and may subsequently use this signal, the common mode interference signal, in the right-leg drive feedback circuit. The output of the RLD amplifier, the inverse of the common mode interference signal, is preferably connected to the common-mode rejection electrode through IN4 to drive the common-mode of the system.

In an embodiment of the present disclosure, the device is configured to measure the Wilson's central terminal. Said measurement may be the result of a signal processing, such as the output provided by the Wilson Reference Generator Circuit, such as when said circuit is provided with the signal of the three ECG electrodes. Preferably the device is configured for continuously measuring the Wilson's central terminal.

In a preferred embodiment of the present disclosure, the device is configured to invert the Wilson's central terminal, such as the measurements of the Wilson's central terminal, and provide it, such as apply it, to the common mode rejection electrode. The device may for example comprise a Wilson Central Terminal based active right leg drive circuit. The common mode rejection electrode may thereby provide a signal to the user that is the inverse of the common mode interference, and consequently actively suppress noise.

In an embodiment of the present disclosure the common mode interference signal is a measured Wilson central terminal, such as by a wilson reference generator circuit. In another embodiment of the present disclosure, the common mode interference signal is a measured common mode, such as by a common mode detector circuit.

For example the right-arm (RA), left-arm (LA), left-leg (LL) and right-leg (RL) electrodes may be connected to an input pin each of the ECG device, such as the IN1, IN2, IN3 and IN4 pins respectively. The ECG device may thereafter use the Common-Mode Detector to measure the common-mode of the system by averaging the voltage of input pins IN1, IN2 and IN3, and uses this signal in the right-leg drive feedback circuit. The output of the RLD amplifier is connected to RL through IN4 to drive the common-mode of the system.

Three electrodes may be placed toward the Right Arm (RA), Left Arm (LA), and Left Leg (LL) and sense the electric potentials from the heart. The ECG leads are derived as the differential signals between two electrodes: Lead I = LA - RA, Lead II = LL - RA, Lead III = LL - LA.

Augmented leads provide enhanced vector information to determine the heart's electrical axis (aVR, aVL, aVF). These are derived via the midpoint of two limbs with respect to the third limb: aVR = RA - (LA + LL) / 2, aVL = LA - (LL+RA) / 2, aVF = LL - (LA + RA) / 2.

In a preferred embodiment of the present disclosure, the device is configured to derive ECG signals from the ECG electrodes to an inverted common-mode interference signal. The common-mode interference signal may thereafter be stored and/or applied to the common mode rejection electrode. Thereby, the common-mode interference signal may be provided back to the user, in order to actively cancel common-mode interference.

In an embodiment of the present disclosure, the ECG signals from the ECG electrodes are connected to the Wilson reference generator circuit, and wherein the Wilson reference generator circuit is configured to generate a common-mode interference signal. Preferably, said common-mode interference signal is the inverse of the common-mode interference and may be derived/obtained by measuring the deviation of the Wilson Central Terminal from the center of the ECG electrodes.

In yet an embodiment of the present disclosure, the inverter stage is configured to invert the common-mode interference signal to the inverted common-mode interference signal. The inverter stage may comprise or consist of an RLD amplifier. Thereby, the inversion of the common mode interference signal may be performed by connecting the common-mode interference signal to the RLD amplifier. Preferably, the output from the inverter stage, such as the RLD amplifier is connected to the common mode rejection electrode, and thereby said output may be applied to the user, such as to the centrally located common mode rejection electrode.

In yet another embodiment of the present disclosure, the inverted common-mode interference signal is connected to the common mode rejection electrode to actively cancel common-mode interference.

*Data processing*

In a further embodiment of the present disclosure, the ECG data comprises or consists of two stored ECG leads, such as Lead I and Lead III. The processing unit of the electrocardiography device may use the ECG signals to produce a number of leads. It is a preference that the ECG data consists of two ECG leads, such as Lead I and Lead III. These leads may be used by the electrocardiography device , and or another processing unit, such as a remote server, to produce additional leads. For example the two leads may be used to produce three standard leads and further a number of augmented leads, for example three augmented leads. It is a preference that the two stored leads are two bipolar leads and that these may be used to produce a third bipolar lead. Furthermore, the two stored bipolar leads may be used to produce a number of augmented leads.

Leads I, II and III are typically called the limb leads. The electrodes that form these signals are normally located on the limbs, one on each arm and one on the left leg. The limb leads form the points of what is known as Einthoven's triangle.

Leads aVR, aVL, and aVF are commonly referred to as the augmented limb leads. They are derived from the same three electrodes as leads I, II, and III, but they use Goldberger's central terminal as their negative pole. Goldberger's central terminal is a combination of inputs from two limb electrodes, with a different combination for each augmented lead. It is referred to immediately below as "the negative pole".

Lead augmented vector right (aVR) has the positive electrode on the right arm. The negative pole is a combination of the left arm electrode and the left leg electrode:

$$aVR = RA - \frac{1}{2}(LA + LL) = \frac{3}{2}(RA - V_W)$$

Lead augmented vector left (aVL) has the positive electrode on the left arm. The negative pole is a combination of the right arm electrode and the left leg electrode:

$$aVL = LA - \frac{1}{2}(RA + LL) = \frac{3}{2}(LA - V_W)$$

Lead augmented vector foot (aVF) has the positive electrode on the left leg. The negative pole is a combination of the right arm electrode and the left arm electrode:

$$aVF = LL - \frac{1}{2}(RA + LA) = \frac{3}{2}(LL - V_W)$$

Together with leads I, II, and III, augmented limb leads aVR, aVL, and aVF form the basis of the hexaxial reference system, which is used to calculate the heart's electrical axis in the frontal plane.

Interpretation of the ECG is ultimately that of pattern recognition. In order to understand the patterns found, it is helpful to understand the theory of what ECGs represent. The theory is rooted in electromagnetics and boils down to the four following points:

- depolarization of the heart towards the positive electrode produces a positive deflection
- depolarization of the heart away from the positive electrode produces a negative deflection
- repolarization of the heart towards the positive electrode produces a negative deflection
- repolarization of the heart away from the positive electrode produces a positive deflection

Thus, the overall direction of depolarization and repolarization produces positive or negative deflection on each lead's trace. For example, depolarizing from right to left would typically produce a positive deflection in lead I if the two vectors point in the same direction. In contrast, that same depolarization would produce minimal deflection in V1 and V2 because the vectors are perpendicular, and this phenomenon is called isoelectric.

Normal rhythm produces four entities - a P wave, a QRS complex, a T wave, and a U wave - that each have a fairly unique pattern.

The P wave represents atrial depolarization.

The QRS complex represents ventricular depolarization.

The T wave represents ventricular repolarization.

The U wave represents papillary muscle repolarization.

Changes in the structure of the heart and its surroundings (including blood composition) change the patterns of these four entities.

The U wave is not typically seen and its absence is generally ignored. Atrial repolarization is typically hidden in the much more prominent QRS complex and normally cannot be seen without additional, specialized electrodes.

In a further embodiment of the present disclosure, the ECG data further comprises time-stamps, such as of registered events. The time stamps may be added to the ECG data and/or during processing of the ECG signals to the ECG data. The time stamps may be added as a result of the electrocardiography device receiving the first user input. Additionally or alternatively, the first user input may lead to ECG data obtained during a specific time interval, such as between a predefined time before receiving the first user input and a predefined time after the receiving the first user input, is transferred to a second memory.

Leads

A "lead" is an angle of looking at the heart. A standard ECG includes 12 leads, i.e. 12 different angles of orientation in regards to the heart. Each lead provides information about different parts of the heart. A standard ECG typically requires 10 electrodes, carefully positioned at their designated location, in order to provide a 12-lead view.

In a preferred embodiment of the present disclosure, the three ECG electrodes are each positioned towards the left arm (VLA), towards the right arm (VRA), and towards the left leg (VLL) respectively. In a further embodiment of the present disclosure, the six leads comprise or consist of the following:

| Name | Signal |
|---|---|
| Lead I | VLA-VRA |
| Lead II | VLL-VRA |
| Lead III | VLL-VLL |
| aVR | VRA-(VLA-VLL)/2 |
| aVL | VLA-(VLL+VRA)/2 |
| aVF | VLL-(VLA+VRA)/2 |

*Electrode positions*

The electrical impulse for contraction of the heart stems from the sinus node (located in the right atrium), which acts as the heart's natural pacemaker. The electrical current is then transmitted via specific pathways throughout the heart, enabling regular contraction and relaxation. This electrical current can be detected on the surface of the body (i.e. the chest wall) via adhesive electrodes.

From the sinus node, the electrical impulse starts by spreading throughout the atria (from right to left). When this happens, the cells lose their internal negativity, a process known as depolarization. The depolarization of the atria causes them to contract.

The electrical current then spreads to the atrioventricular node (AV node), from where it is further transmitted to the intraventricular Septum (separates the left and right ventricles). In order to depolarize the ventricles, the electrical impulse travels through the bundle of His, along the right and left bundle branches (from left to right), and ends at the Purkinje fibers. This process causes depolarization of the ventricles, causing them to contract.

While the ventricles are being depolarized, the atria are regaining their internal electrical negativity, a process known as repolarization. This allows them to relax. Once the ventricles have fully depolarized, they too become repolarized, and which point they relax as well.

The entire process of depolarization and repolarization is depicted on the ECG. The individual events are represented as spikes and waves, each representing a specific part of the cardiac conduction cycle. This visual representation of the conduction system makes it possible to analyze the heart's electrical activity.
In a further embodiment of the present disclosure, each ECG electrode is configured for detachably connecting to an electrode pad.

In a further embodiment of the present disclosure, the electrocardiography device is configured such that the ECG electrodes can be positioned, when in use, symmetrically across the sternum. The distances and angles between the ECG electrodes may thereby be configured such that said ECG electrodes may be positioned, when in use, symmetrically across the sternum of the user.

In a further embodiment of the present disclosure, the ECG electrodes are located in a substantially equilateral triangular pattern. The ECG electrodes may be located in substantially the same plane, preferably a first plane formed parallel to the first side of the electrocardiography device . The ECG electrodes may form a pattern, along the first side, wherein the distance between each electrode is substantially identical. The ECG electrodes may further form a pattern, along the first plane, wherein the angle between each ECG electrode is equal, i.e. an equilateral triangle for the case of three ECG electrodes. In a further embodiment of the present disclosure two of the sides of the pattern formed by the ECG electrodes are of the same length. The pattern may thereby be an isosceles triangle.

In a further embodiment of the present disclosure, the distance between the ECG electrodes are less than around 15 cm. It is a preference that the electrocardiography device is small such that said electrocardiography device is portable and can easily adhere to the skin of the user for an extended time without discomfort to said user. In a preferred embodiment the distance between each ECG electrode is less than 15 centimeters, more preferably less than 12 centimeters, yet more preferably less than 8 centimeters. The size of the electrocardiography device may depend on the size of the user. For example adults may be provided with an electrocardiography device that is larger than an electrocardiography device that is intended for being used by children.

In a further embodiment of the present disclosure, the device is waterproof, for example according to at least IP54, such as IP67. The electrocardiography device may thereby comprise a waterproof enclosure design. It is a further preference that the electrocardiography device can withstand being used while swimming.

In a further embodiment of the present disclosure, the first side of the housing has a substantially triangular shape, and wherein the three ECG electrodes are located in each corner of said first side. In yet a further embodiment of the present disclosure, the first side of the housing has a substantially three bladed shape, and wherein the three ECG electrodes are located in each distal endpoint of said blades. For example, the first side of the housing may have a shape resembling a three bladed propeller, or three bladed boomerang. Said housing may have any shape that has blades extending outwards from a central point. Said blades may furthermore have any shape, regular and/or irregular.

In another embodiment of the present disclosure, the first side of the housing has a substantially rectangular shape. While it is preferred in specific embodiments of the present disclosure that the ECG electrodes are arranged in a triangular pattern, the housing may be provided in any shape. In such an instance, the ECG pads may be provided in a configuration such that they allow for the arrangement of ECG electrodes to form a triangular pattern. For example the ECG pads may be provided with electrical connections to a housing that is remote from one or more of the ECG electrodes. In a preferred embodiment of the present disclosure, the ECG device comprises a single ECG pad comprising ECG electrodes that are arranged such that they form a triangular pattern. Said ECG pad and the ECG housing may be configured to form one or more electrical connections. For example the housing may comprise a number of exposed electrical contacts. The ECG pad may further comprise wirings from the electrodes, such as the ECG electrodes and/or the common mode rejection electrode, to one or more connectors of the ECG pad. The one or more connectors of the ECG electrode may be configured to form an electrical connection with the one or more electrical contacts of the housing. Preferably each electrical is electrically connected to one connector of the ECG pad, and the housing configured to form an electrical connection to each connector of the ECG pad. Preferably, the electrical connectors of the ECG pad are located in a central location of the ECG pad, thereby the ECG housing may comprise a bundled connector, configured for connecting to all electrodes of the ECG pad. It is a preference that the ECG housing is held in place by the connection formed to the ECG pad, thereby the adhesion of the ECG pad to the skin may not only hold the ECG pad in place but furthermore the ECG housing.

In a further embodiment of the present disclosure, the ECG electrodes are configured such that when in use, the L electrode (left arm electrode) is positioned at a left interspace, the R electrode (right arm electrode) is positioned at a right interspace, the F electrode (foot electrode) is positioned centrally at the sternum. In a specific embodiment of the present disclosure, the L electrode is positioned in the second left interspace, the R electrode in the second right interspace and the F electrode centrally near or below the xiphoid process. In yet a further embodiment of the present disclosure, the L electrode is positioned in the third left interspace, the R electrode in the third right interspace and the F electrode centrally near or below the xiphoid process. In yet a further embodiment of the present disclosure, the L electrode is positioned in the fourth left interspace, the R electrode in the fourth right interspace and the F electrode centrally near or below the xiphoid process.

*User indication*

In a further embodiment of the present disclosure, the device comprises means for indication of battery status and/or lead-off, such as one or more of the electrodes not being properly attached to the skin of the user. Said means may comprise or consist of a means for providing a visual signal, such as at least one LED. Said means may additionally or alternatively comprise a speaker for providing an audible signal to the user. Said means may additionally or alternatively comprise means for providing a vibrating alert to the user. Different types of signals, such as different light colors and/or different sounds may be used for notifying the user about different events, such as the battery status, the storage status of the first and/or second memory, a lead-off of any of the electrodes.

*Method*

In a second aspect, the present invention relates to a method for electrocardiographic measurements of an user. Preferably the method comprises the provision of a portable electrocardiography device. Said portable electrocardiography device may comprise an arrangement of electrodes located on a first plane defined by a first side of a housing of said electrocardiography device . Furthermore, said arrangement of electrodes may consist or comprise of a number of electrodes such as three or four. In a preferred embodiment of the present disclosure, the arrangement of electrodes consists of three ECG electrodes and one Common mode rejection electrode. It is a further preference that the ECG electrodes are located on the first side of the housing of the electrocardiography device . It is a further preference that the three ECG electrodes are located in a triangular pattern. In yet a preferred embodiment of the present disclosure, the electrocardiography device comprises a Common mode rejection electrode that is located in the same plane as the ECG electrodes. In a further preferred embodiment of the present disclosure the Common mode rejection electrode is located substantially in the center of the triangular pattern formed by the ECG electrodes. While the ECG electrodes may form a triangular pattern, it should be noted that the housing may not necessarily be provided in a similar shape. Instead it may be a preference that the housing is provided in a shape that minimizes the area of the first side, and/or minimizes the projected area of the electrocardiography device onto a surface parallel with the first surface. Thereby, the device may be provided in for example a substantially three bladed shape, such as three bladed boomerang and/or a three bladed propeller. The housing of the electrocardiography device may thereby have three arms extending outwards from the centrally located Common mode rejection electrode. Each arm of said three arms may have any length independent of the other arms, and may further extend outward from the central part of the housing at any angle. The special case being wherein each arm forms a 60 degree angle to the adjacent arm, and wherein the length of each arm is identical. In this configuration the pattern of the electrodes may form an equilateral triangle.

In a preferred embodiment of the present disclosure, the method comprises the step of positioning said portable electrocardiography device on the chest of the user. By positioning the electrocardiography device on the chest of the user, adhesive electrode pads, located on each electrode, may adhere to the surface of the skin of the user. The adhesive strength provided of said electrode pads are preferably sufficient to hold the electrocardiography device in place. It is a preference that the electrocardiography device is positioned such that the ECG electrodes are symmetrically located across the chest of the user.

In yet an embodiment of the present disclosure, the method comprises the step of acquiring ECG signals from the electrodes, such as the three ECG electrodes and the Common mode rejection electrode. The acquisition of ECG signals may be enabled, at least in part, by the use of a processing unit that is in electrical contact with each of the electrodes. Said processing unit may advantageously be configured for processing of the ECG signals into ECG data. Preferably, the electrocardiography device is configured to store said ECG data on a first and/or second memory. It should be noted that the electrocardiography device may be configured in various ways while being able to carry out the presently disclosed method. In a preferred embodiment of the present disclosure, the portable electrocardiography device is defined as described elsewhere herein.

In a further embodiment of the present disclosure, the ECG electrodes are configured such that when in use, the L electrode (left arm electrode) is positioned at a left interspace, the R electrode (right arm electrode) is positioned at a right interspace, the F electrode (foot electrode) is positioned centrally at the sternum. In a specific embodiment of the present disclosure, the L electrode is positioned in the second left interspace, the R electrode in the second right interspace and the F electrode centrally near or below the xiphoid process. In yet a further embodiment of the present disclosure, the L electrode is positioned in the third left interspace, the R electrode in the third right interspace and the F electrode centrally near or below the xiphoid process. In yet a further embodiment of the present disclosure, the L electrode is positioned in the fourth left interspace, the R electrode in the fourth right interspace and the F electrode centrally near or below the xiphoid process.

In a further embodiment of the present disclosure, the distance between the ECG electrodes are less than around 15 cm. It is a preference that the electrocardiography device is small such that said electrocardiography device is portable and can easily adhere to the skin of the user for an extended time without discomfort to said user. In a preferred embodiment the distance between each ECG electrode is less than 15 centimeters, more preferably less than 12 centimeters, yet more preferably less than 8 centimeters. The size of the electrocardiography device may depend on the size of the user. For example adults may be provided with an electrocardiography device that is larger than an electrocardiography device that is intended for being used by children.

In a further embodiment of the present disclosure, the ECG electrodes are located in a substantially equilateral triangular pattern. The ECG electrodes may be located in substantially the same plane, preferably a first plane formed parallel to the first side of the electrocardiography device . The ECG electrodes may form a pattern, along the first side, wherein the distance between each electrode is substantially identical. The ECG electrodes may further form a pattern, along the first plane, wherein the angle between each ECG electrode is equal, i.e. an equilateral triangle for the case of three ECG electrodes.

In a further embodiment of the present disclosure, the electrocardiography device is configured such that the ECG electrodes can be positioned, when in use, symmetrically across the sternum. The distances and angles between the ECG electrodes may thereby be configured such that said ECG electrodes may be positioned, when in use, symmetrically across the sternum of the user.

*Items*

1. A portable electrocardiography device comprising:

- a housing;
- a battery;
- an arrangement of electrodes located on a first plane defined by a first side of the housing, including:

    - three ECG electrodes arranged in a triangular pattern;
    - a common mode rejection electrode located in the center of the triangular pattern; and

- a processing unit in electrical communication with the electrodes, said processing unit configured for receiving an ECG signal from each electrode and processing the ECG signals into ECG data.

*Communication means*

2. The portable electrocardiography device according to any one of the preceding items, wherein said device comprises means for wireless communication, such as 4G LTE, 5G and/or Bluetooth Low Energy.

3. The portable electrocardiography device according to any one of the preceding items, wherein said device comprises means for wireless communication with a cloud-based server and/or a personal computing device, such as a smartphone.

4. The portable electrocardiography device according to any one of the preceding items, wherein the device is configured to, upon receiving a first user input, store ECG data and/or ECG signals obtained during a recording interval, said recording interval being defined by a predetermined time before receiving said first user input and a predetermined time after receiving said first user input, such as on a second memory.

5. The portable electrocardiography device according to any one of the preceding items, wherein said ECG data and/or ECG signals are stored on a second memory of said portable electrocardiography device.

6. The portable electrocardiography device according to any one of the preceding items, wherein said device is configured to, upon receiving a second user input, continuously transmit ECG data to the personal computing device, such as low-resolution ECG data for ensuring correct placement of the electrodes.

7. The portable electrocardiography device according to any one of the preceding items, wherein said device is configured to periodically, such as upon recharging of the battery, transmit stored ECG data, such as from the secondary memory, to the cloud-based server.

*Electrical components*

8. The portable electrocardiography device according to any one of the preceding items, wherein the device is configured to measure the Wilson's central terminal, such as continuously measure.

9. The portable electrocardiography device according to any one of the preceding items, wherein the device is configured to invert the Wilson's central terminal and apply it to the common mode rejection electrode.

10. The portable electrocardiography device according to any one of the preceding items, wherein the device is configured to derive ECG signals from the ECG electrodes to an inverted common-mode interference signal, wherein the inverted common-mode interference signal is applied to the common mode rejection electrode.

11. The portable electrocardiography device according to item 10, comprising a Wilson reference generator circuit, wherein the ECG signals from the ECG electrodes are connected to the Wilson reference generator circuit, and wherein the Wilson reference generator circuit is configured to a common-mode interference signal.

12. The portable electrocardiography device according to item 11, further comprising an inverter stage, wherein the inverter stage is configured to invert the common-mode interference signal to the inverted common-mode interference signal.

13. The portable electrocardiography device according to any one of items 10-12, wherein the inverted common-mode interference signal is connected to the common mode rejection electrode to actively cancel common-mode interference.

14. The portable electrocardiography device according to any one of the preceding items, wherein the device is configured for continuously receiving ECG signals and/or processing ECG signals into ECG data.

15. The portable electrocardiography device according to any one of the preceding items, wherein the device is configured to automatically and continuously receive and/or process ECG signals upon connecting each ECG electrode to an electrode pad.

16. The portable electrocardiography device according to any one of the preceding items, wherein the device comprises a first memory for loop recording of ECG data.

17. The portable electrocardiography device according to any one of the preceding items, wherein the device is configured for loop recording of ECG data, such as on the first memory.

18. The portable electrocardiography device according to any one of the preceding items, wherein the device is configured to receive the first user input from the personal computation device and/or input means located on said electrocardiography device, such as a button.

19. The portable electrocardiography device according to any one of the preceding items, wherein the electrocardiography device is capable of being used at least 4 days without replacing or recharging the battery.

*Data processing*

20. The portable electrocardiography device according to any one of the preceding items, wherein the ECG data comprises or consists of two stored ECG leads, such as Lead I and Lead III.
21. The portable electrocardiography device according to any one of the preceding items, wherein the ECG data further comprises time-stamps, such as of registered events.
22. The portable electrocardiography device according to any one of the preceding items, wherein the two stored ECG leads are selected such that they can be used to calculate six ECG leads.
23. The portable electrocardiography device according to any one of the preceding items, wherein the six leads comprise or consist the following:

| Lead I | VLA-VRA |
|--------|---------|
| Lead II | VLL-VRA |
| Lead III | VLL-VLA |
| aVR | VRA-(VLA-VLL)/2 |
| aVL | VLA-(VLL+VRA)/2 |
| aVF | VLL-(VLA+VRA)/2 |

*Electrode positions*

24. The portable electrocardiography device according to any one of the preceding items, wherein each ECG electrode is configured for detachably connecting to an electrode pad.
25. The portable electrocardiography device according to any one of the preceding items, wherein the device is configured to, when in use, be positioned symmetrically across the sternum.
26. The portable electrocardiography device according to any one of the preceding items, wherein the ECG electrodes are located in a substantially equilateral triangular pattern.
27. The portable electrocardiography device according to any one of the preceding items, wherein the distance between the ECG electrodes are less than around 15 cm
28. The portable electrocardiography device according to any one of the preceding items, wherein said device is waterproof.
29. The portable electrocardiography device according to any one of the preceding items, wherein the first side of the housing has a substantially triangular shape, and wherein the three ECG electrodes are located in each corner of said first side.
30. The portable electrocardiography device according to any one of the preceding items, wherein the ECG electrodes are configured such that they may be positioned, respectively, at the locations defined in the table below:

| LA(L) | second left interspace |
|-------|------------------------|

(continued)

| | |
|---|---|
| RA(R) | second right interspace |
| LL(F) | below the xiphoid process |

*User indication*

31. The portable electrocardiography device according to any one of the preceding items, wherein said device comprises means for indication of battery status and/or lead-off, such as at least one LED.

32. A method for electrocardiographic measurements of an user, comprising:

- providing a portable electrocardiography device comprising an arrangement of electrodes located on a first plane defined by a first side of the housing, including:

  i. three ECG electrodes arranged in a triangular pattern; and
  ii. a common mode rejection electrode located in the center of the triangular pattern;

- positioning said portable electrocardiography device on the chest of said user; and
- acquiring ECG signals from said electrodes;

thereby performing electrocardiographic measurements of the user.

33. The method according to item 32, wherein the portable electrocardiography device is defined according to any of item 1-31.

34. The method according to any one of items 32-33, wherein the electrodes of the portable electrocardiography device are positioned, on said user, respectively, at the second left interspace, the second right interspace and at or below the xiphoid process.

35. The method according to any one of items 32-34, wherein the electrodes are positioned, on said subject, with a spacing between any one of the electrodes that is less than around 15 cm.

36. The method according to any one of items 32-35, wherein the electrodes are positioned, on said subject, according to an equilateral triangle.

37. The method according to any one of items 32-36, wherein the electrodes are positioned, on said subject, symmetrically across the sternum.

**Detailed description of drawings**

The invention will in the following be described in greater detail with reference to the accompanying drawings. The drawings are exemplary and are intended to illustrate some of the features of the presently disclosed electrocardiography device or method of use thereof, and are not to be construed as limiting to the presently disclosed invention.

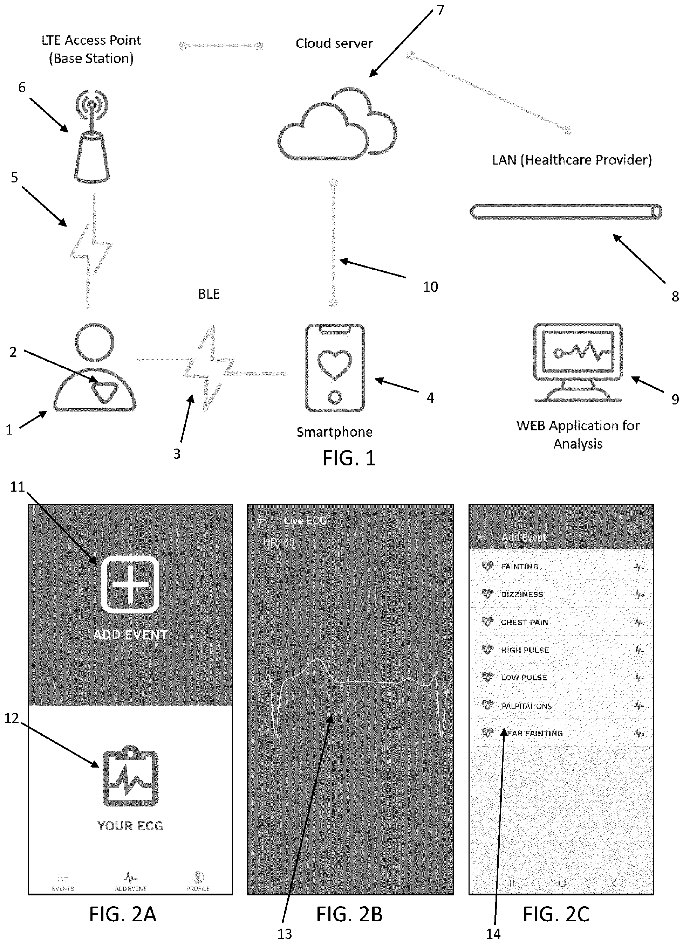

Fig. 1 shows an infrastructure for accurate ECG measurement by a portable electrocardiography device. A user (1) may be provided by for example a medical professional with the portable ECG device (2) and instructed to wear said device for an extended amount of time. The ECG device may be attached to the chest area of the user by the medical professional. Said ECG device may comprise one or more adhesive ECG pads, for providing electrical contacts with the user and for adhering to the user. Thereby, the attachment may be facilitated by the one or more ECG pads. The ECG pad may for example be provided as a single unit comprising three electrodes and further configured for forming electrical connections to contacts on the housing. Preferably the ECG device is attached to the chest area of the user such that the ECG electrodes are positioned symmetrically across the sternum of the user. For example electrode L (LA) may be positioned on the left side of the sternum, in an interstitial space. Meanwhile electrode R (RA) may be positioned at the same interstitial space, but on the right hand side. For example the L and R electrode may be positioned in the third left interstitial space and third right interstitial space respectively. At the same time the electrode F (LL) may be positioned centrally over the sternum, for example near or below the xiphoid process. The ECG device may be configured such that the ECG electrodes form an equilateral triangle, given that each side of the triangular pattern formed by said ECG electrodes have the same distance. The device, such as the one or more electrode pad, may further comprise a common mode rejection (CMR) electrode for registering, detection and/or storing of background noise data. The ECG device may comprise means for communication with electrical devices. For example means for (proximal) communication (3) with a proximal personal computation unit (4). The (proximal) means for communication may for example comprise or consist of a Bluetooth chip, for transmitting and receiving communication with a personal computational device (4) having a capability of communication over Bluetooth. Said personal computational device may for example be a smartphone. It is a further preference that at least part of the ECG data is transmitted to the proximal computational device, for subsequently being presented on said personal computational device, to the user. Furthermore, the ECG device preferably comprises means for communication with a remote server (5), such as means for communication over mobile networks, such as LTE Cat M1. The ECG device may thereby communicate with an LTE Access point, such as a base station (6), that in turn is connected to a remote server (7), i.e. a cloud server. It should be noted that the ECG device may alternatively or additionally comprise means for communication with the remote server (10). This arrangement thereby allows the ECG device to communicate with the remote server, and transmit ECG data to said remote server. Preferably stored ECG data, such as from a second memory unit of the ECG device may thereby be transmitted to the remote server. The stored ECG data may comprise or consists of a number of leads, preferably two leads, such as Lead I and Lead III, in addition to event data, that subsequently can be used, by the remote server, or a further computational device, for processing of the ECG data in order to obtain a number of additional leads. Preferably the remote server is configured to obtain three bipolar leads, such as Lead I, II, and III and additionally three augmented leads, such as aVR, aVL, and aVF. The remote server may be connected to a LAN of a healthcare provider (8). Healthcare providers may thereafter be provided with the ability to access said data, for example by the use of a WEB application (9), wherein said healthcare provider/medical professional is able to browse and analyze the ECG data. The data may for example be visualized together with annotations of arrhythmias found by the WEB application analysis software. It may be a preference that the ECG device is configured such that said device transmits ECG data to the personal computation device, over for example BLE, upon receiving a second input from a user. Said second input may for example be a Bluetooth pairing between the personal computation device and the ECG device, or the user providing an input to the personal computation device and/or the ECG device, such as a press of a button or touchscreen. The ECG device may be configured such that upon receiving the second input, the ECG device transmits at least part of the ECG data, such as Lead I and Lead II, to the personal computation device. The user may be presented with a visual representation of said transmitted ECG data, such as one or more electrocardiograms. The user may use this information in order to check that the electrodes are correctly positioned, or a health professional may use said information in order to draw conclusions about a suspected condition of the user. The ECG device may be configured to periodically transmit ECG data to the remote server, such as when said ECG device is placed in charging mode, wherein the rechargeable electrical batteries of the ECG device are being recharged. Alternatively, or additionally, the ECG device may be configured to communicate with a remote server through the personal computation device. This may be enabled by using the personal computation device for relaying information to the remote server. ECG data may for example be transmitted to the personal computation device over BLE, and thereafter said data may be temporarily stored, before being transmitted to the remote server over a mobile network.

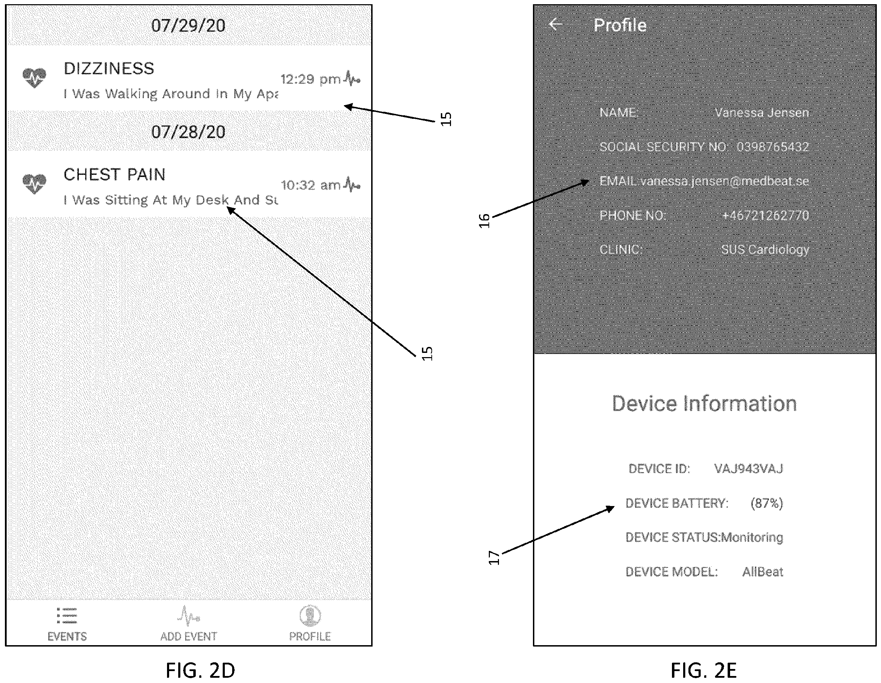

Fig. 2 shows a number of user interfaces of a smartphone app for obtaining status of the ECG device and registering of abnormal events. Fig. 2A shows a start screen which may be presented to the user upon opening of the smartphone app. The start screen may present the user with a number of links, such as to an event screen (11), an ECG screen (12), and potentially to additional user screens. During the initiation of a recording, the user may want to ensure that the electrodes are working properly. The user may enter the ECG screen wherein the user is presented with a graphical representation of the ECG data, Fig. 2B. In the present example, said graphical representation shows only a single lead (13). The purpose may be to provide the user with a simple overview that still allows to ensure that the ECG device is able to measure ECG signals properly. For this purpose there may not be a need to display multiple leads to the user. As disclosed elsewhere herein, the ECG device may start transmitting ECG data, such as the mentioned single lead, to the smartphone, or other type of computational device, upon receiving a second user input. Said second user input may for example be provided by the smartphone upon the user entering the testing interface, wherein the user is to be presented with the at least one lead. Said ECG screen may alternatively or additionally be presented to the user of the ECG device upon providing the ECG device with a second input, such as pairing of the ECG device and the personal computation device. Alternatively, or additionally the second user input may be a signal generated by the press of a button or a touch screen, on the ECG device and/or the personal computation device. Upon receiving the second input, the ECG device transmits at least part of ECG data to the personal computation device. The ECG data transmitted to the personal computation device may be down sampled, and may thereby have a lower resolution. Said transmitted ECG data may comprise the momentarily obtained ECG data, such as the momentarily processed ECG signals. The user interface may provide the user with a graphical representation of a number of the leads, preferably at least two. In that case the personal computation device may not be required to compute additional leads. For the remote server, it may however be a preference that said server, or another computational device connected thereto, has the capabilities to compute additional leads, than the leads transmitted to the remote server, such as Lead I and Lead III. Thereby, the remote server, or the computational device connected thereto may be configured to compute bipolar leads, e.g. Lead I, II, and III, and additionally a number of augmented leads such as aVL, aVF, aVR.

Further, the home screen may allow the user to enter the event screen, Fig. 2C, wherein the user may be provided with the ability to register events (14), for example by selecting any of a number of predefined events. The events and/or the predefined events may comprise for example fainting, dizziness, chest pain, high pulse, low pulse, palpitation, and/or near fainting. Upon registering of an event the ECG device may be configured to add time points of said registered event to a memory of the ECG device, such as the first memory. Additionally, or alternatively the registering of an event may lead to an interval of the ECG data being transferred from a first memory onto a secondary memory, such as from a first memory unit to a secondary memory unit. The transferred ECG data may comprise ECG data obtained by the ECG device during an interval comprising the time point for registering of the event. For example the interval may be between a predefined time before the registering of the event and a predefined time after the registering of the event. The transferred ECG data may comprise the Leads, such as Leads I and III obtained during this interval, in addition to additional information, event information, provided during registering of the event, such as the time point of the event, the type of event, text-based comments about the event, and/or audio recordings about the event. Said event information may comprise information related to said events, such as information related to what the user was experiencing when said user noticed the abnormal event, what activity said user was carrying out, and/or speculations of the cause of the event.

Additionally, the smartphone app may comprise a screen wherein registered events are presented to the user, a registered event screen, Fig. 2D. This screen may be configured such that the user is capable of accessing, editing and/or modifying information that is connected to each registered event, in addition to accessing the recordings of each event, for example by selecting one of the registered events (15). Further, the smartphone app may comprise an information screen, Fig. 2E, wherein the user is provided with registered information related to the current profile (16), i.e. information about the present user of the ECG device, such as the name, social security number, and contact info, and device information (17), such as battery status, device model and memory status.

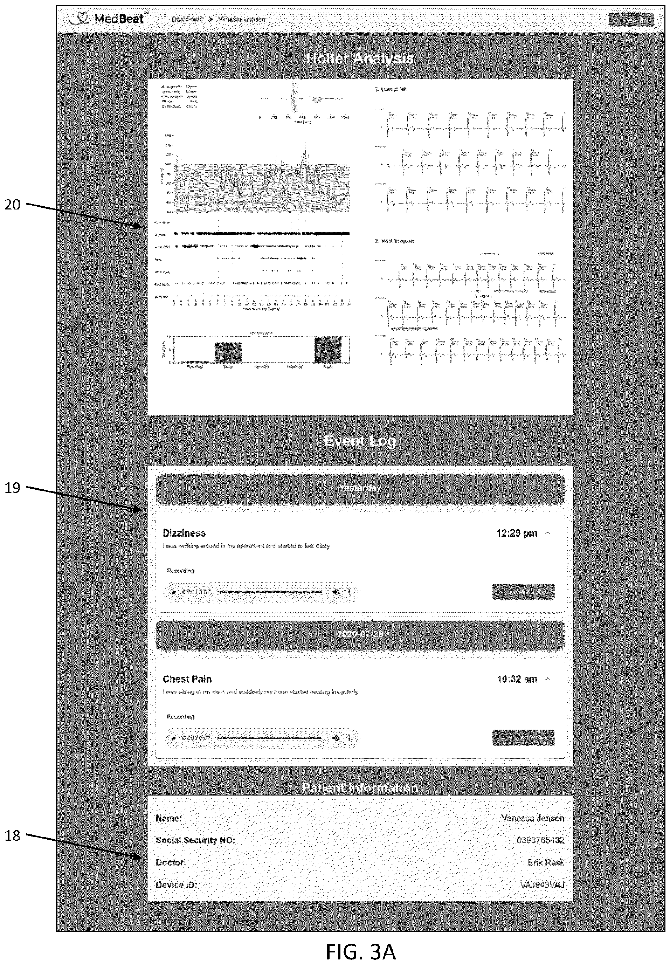

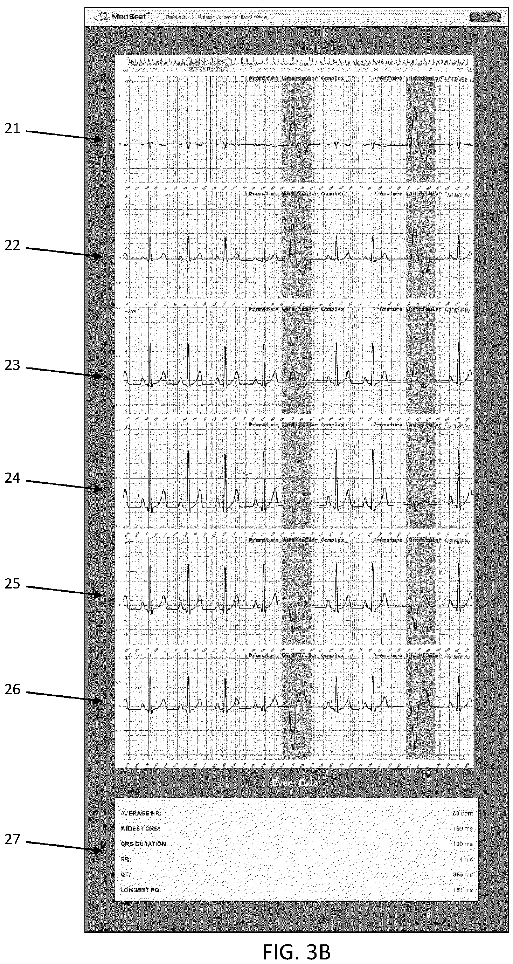

Fig. 3 shows a user interface of a web application for use by a medical professional comprising ECG data obtained during an extended amount of time. Fig. 3A shows an initial interface providing an overview of information about the user (18), and events registered by the user, i.e. an event log (19), together with a Holter analysis (20). The medical professional may for example be provided with the name, the social security number, the doctor, the type of device of said user. Furthermore, the interface may allow the medical professional to access each registered event, such as at least part of the associated ECG data. This may be presented to the medical professional in a secondary interface, Fig. 3B, wherein multiple leads are presented, preferably six leads, such as Leads I (21), Lead II (22), Lead III (23), augmented lead aVR (24), augmented lead aVF (25), and augmented lead aVL (26). The remote server may have processed the ECG data comprising typically two leads into six leads. Additional filters and data optimizations may further have been applied to the ECG data. This information is provided in the secondary interface to the medical professional who can identify any abnormalities related to the electrical activity of the heart, such that conclusions may be drawn about the suspected medical conditions of the user. Additional statistics about each event are preferably present in the same secondary screen, such as the average heart rate, the longest QRS complex, the average QRS complex interval, the average RR interval, the average QT interval and the longest PQ interval (27).

Fig. 4 shows a schematic illustration of the ECG device, shown from the first side, comprising an ECG pad (28) and a housing (29). The ECG device here comprises a single ECG pad wherein the ECG pad comprises both the ECG electrodes (30) and the common mode rejection electrode (31). In a specific embodiment of the present disclosure, the housing comprises a number of electrical contact points (32) for providing electrical signals to the electrical circuitry of the ECG housing. Electrical connectors (33) may be provided as part of the ECG pad for forming electrical connections between the electrodes, such as the ECG electrodes and/or the common mode rejection electrode, and the electrical contact points of the housing (32). The ECG pad is here shown as semitransparent for illustrative purposes, thereby showing a typical arrangement of the ECG pad and the ECG housing. The first side of the electrocardiography device (34) is the side that is to face the user, during use. The first side of the electrocardiography device may coincide with the first side of the housing, if the housing comprises the electrodes. However, if the electrodes are provided as part of a separate ECG pad, said electrodes may be positioned between the ECG housing and the user.

Fig. 5 shows block diagrams describing the electrical circuitry of the ECG device for processing of the ECG signal. Among other features of the circuit, the processing of the signal to acquire the right leg drive. The common mode rejection electrode may be driven by the RLD to counter any differences between the reference voltage and the detected common-mode level. Thereby acting to reduce the amount of power-line common-mode interference. The figure shows a number of inputs (IN1-IN6) that are filtered by an electromagnetic interference filter (EMI). Typically, only three in-channels are used, such as IN1, IN2, and IN3, each being connected to an electrode and thereby providing the ECG signal of said each electrode, for example IN1 may be connected to RA, IN2 may be connected to LA, and IN3 may be connected to LL. Thereby each in-channel is provided the ECG signal from each respective ECG electrode. The in-channels (IN1, IN2, and IN3) may be used in a driven right leg circuit in order to eliminate measurement noise by actively cancelling the common-mode interference. These in signals are thereby used to derive an inverted signal of the common-mode interference. The in signals are provided to a Common-Mode Detector to measure the common-mode of the system by averaging the voltage of input pins IN1, IN2 and IN3, and uses this signal in the right-leg drive feedback circuit. The output of the RLD amplifier is connected to RL through IN4 to drive the common-mode of the system. Wilson reference generator circuit. First, said inputs are routed to a Wilson reference electrode amplifier that filters out the common mode interference. This may for example be the sum of Lead I, Lead III, -Lead II. Given the conventional notation, a non-zero sum of the above equation is expected to represent the common mode interference. By routing the output of the Wilson reference electrode amplifier (Wilson ref.) through the Wilson reference output (WCT), to the Right-leg drive amplifier negative input (RLDINV), the RLD amplifier (RLD Amp.) produces an inverted common mode interference signal that is output to the Right-leg drive amplifier output (RLDOUT). This inverted common mode interference signal is thereafter connected to the common mode rejection electrode for actively cancelling of the common-mode interference.

## Claims

1.  A portable electrocardiography device comprising:

    • a housing;
    • a battery;
    • an arrangement of electrodes located on a first plane defined by a first side of the portable electrocardiography device, including:

        • three ECG electrodes arranged in a triangular pattern;
        • a common mode rejection electrode located in the center of said triangular pattern; and

    • a processing unit in electrical communication with the electrodes, said processing unit configured for receiving an ECG signal from each electrode and processing the ECG signals into ECG data.

2.  The portable electrocardiography device according to claim 1, wherein the device is configured to derive ECG signals from the ECG electrodes to an inverted common-mode interference signal, wherein the inverted common-mode interference signal is applied to the common mode rejection electrode.

3.  The portable electrocardiography device according to claim 2, comprising a Common-mode detector circuit, wherein the ECG signals from the ECG electrodes are connected to the Common-mode detector circuit, and wherein the Common-mode detector circuit is configured to output a common-mode interference signal.

4.  The portable electrocardiography device according to claim 3, further comprising an inverter stage, wherein the inverter stage is configured to invert the common-mode interference signal to the inverted common-mode interference signal.

5.  The portable electrocardiography device according to any one of claims 2-4, wherein the inverted common-mode interference signal is connected to the common mode rejection electrode to actively cancel common-mode interference.

6.  The portable electrocardiography device according to any one of the preceding claims, wherein the electrocardiography device is configured to, when in use, be positioned symmetrically across the sternum.

7.  The portable electrocardiography device according to any one of the preceding claims, wherein the ECG electrodes are arranged in a substantially equilateral triangular pattern.

8.  The portable electrocardiography device according to any one of the preceding claims, wherein the distance between any of the ECG electrodes is less than 15 cm.

9.  The portable electrocardiography device according to any one of the preceding claims, wherein said electrocardiography device is configured such that the housing is fixed in position, with respect to the user, by a connection to at least one ECG pad.

10. The portable electrocardiography device according to any one of the preceding claims, wherein the position of the ECG electrodes and the common mode rejection electrode are fixed with respect to the ECG housing.

11. The portable electrocardiography device according to any one of the preceding claims, wherein said device comprises means for communication over mobile networks and Bluetooth.

12. The portable electrocardiography device according to any one of the preceding claims, wherein the housing has a first side for facing the user during use, said first side of the housing having a substantially triangular shape, and wherein the three ECG electrodes are located in each corner of said first side of the housing.

13. A method for electrocardiographic measurements of a user, comprising:

• providing a portable electrocardiography device comprising an arrangement of electrodes located on a first plane defined by a first side of the housing, including:

i. three ECG electrodes arranged in a triangular pattern; and
ii. a common mode rejection electrode located in the center of the triangular pattern;

• positioning said portable electrocardiography device on the chest of said user; and
• acquiring ECG signals from said electrodes;

thereby performing electrocardiographic measurements of the user.

14. The method according to claim 13, wherein the portable electrocardiography device is defined according to any one of claims 1-12.

15. The method according to any of claims 13-14, wherein the ECG device is positioned such that the ECG electrodes attach symmetrically across the sternum of the user at: the second left interspace, the second right interspace, and below the xiphoid process, respectively.

FIG. 1

FIG. 2A

FIG. 2B

FIG. 2C

## FIG. 2D

07/29/20

**DIZZINESS**
I Was Walking Around in My Apa    12:29 pm

07/28/20

**CHEST PAIN**
I Was Sitting At My Desk And St    10:32 am

EVENTS   ADD EVENT   PROFILE

15
15
16
17

## FIG. 2E

← Profile

NAME:  Vanessa Jensen

SOCIAL SECURITY NO: 039876543 2

EMAIL: vanessa.jensen@medbeat.se

PHONE NO: +46721262770

CLINIC:-  SUS Cardiology

**Device Information**

DEVICE ID:  VAJ943VAJ

DEVICE BATTERY:  (87%)

DEVICE STATUS:Monitoring

DEVICE MODEL:  AllBeat

FIG. 3A

FIG. 3B

FIG. 4

FIG. 5

Europäisches
Patentamt

European
Patent Office

Office européen
des brevets

# EUROPEAN SEARCH REPORT

Application Number

EP 20 18 9718

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/208076 A1 (FONG SHANNON [US] ET AL) 25 August 2011 (2011-08-25)<br>* paragraphs [0050] - [0063] *<br>* figures 1-4,12a,12b * | 1-15 | INV.<br>A61B5/0432<br>A61B5/0404<br>A61B5/0428<br>A61B5/0408 |
| A | US 2014/135638 A1 (LISOGURSKI DANIEL M [US] ET AL) 15 May 2014 (2014-05-15)<br>* paragraphs [0050] - [0052] *<br>* paragraphs [0105] - [0110] *<br>* figures * | 1-15 | A61B5/00 |
| A | US 2020/155072 A1 (SZUMANSKI THOMAS [US]) 21 May 2020 (2020-05-21)<br>* the whole document * | 1-15 | |
| A | WO 00/42904 A1 (DEL MAR AVIONICS [US]; DEL MAR BRUCE E [US])<br>27 July 2000 (2000-07-27)<br>* page 9 - page 11; figures 1-4 * | 1-15 | |

TECHNICAL FIELDS
SEARCHED    (IPC)

A61B

The present search report has been drawn up for all claims

| Place of search | Date of completion of the search | Examiner |
|---|---|---|
| The Hague | 2 November 2020 | Bataille, Frédéric |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 20 18 9718

This annex lists the patent family members relating to the patent documents cited in the above-mentioned European search report.
The members are as contained in the European Patent Office EDP file on
The European Patent Office is in no way liable for these particulars which are merely given for the purpose of information.

02-11-2020

| Patent document cited in search report | | Publication date | Patent family member(s) | | Publication date |
|---|---|---|---|---|---|
| US 2011208076 | A1 | 25-08-2011 | BR | PI0906154 A2 | 21-06-2016 |
| | | | CN | 101965150 A | 02-02-2011 |
| | | | EP | 2262420 A1 | 22-12-2010 |
| | | | JP | 5517305 B2 | 11-06-2014 |
| | | | JP | 2011513019 A | 28-04-2011 |
| | | | US | 2011208076 A1 | 25-08-2011 |
| | | | WO | 2009112973 A1 | 17-09-2009 |
| US 2014135638 | A1 | 15-05-2014 | EP | 1690565 A2 | 16-08-2006 |
| | | | JP | 5129453 B2 | 30-01-2013 |
| | | | JP | 5587373 B2 | 10-09-2014 |
| | | | JP | 2006218304 A | 24-08-2006 |
| | | | JP | 2012161625 A | 30-08-2012 |
| | | | JP | 2012196581 A | 18-10-2012 |
| | | | JP | 2014087690 A | 15-05-2014 |
| | | | US | 2006178706 A1 | 10-08-2006 |
| | | | US | 2012203296 A1 | 09-08-2012 |
| | | | US | 2014135638 A1 | 15-05-2014 |
| US 2020155072 | A1 | 21-05-2020 | CN | 111195121 A | 26-05-2020 |
| | | | US | 2020155072 A1 | 21-05-2020 |
| WO 0042904 | A1 | 27-07-2000 | US | 6117077 A | 12-09-2000 |
| | | | WO | 0042904 A1 | 27-07-2000 |

EPO FORM P0459

For more details about this annex : see Official Journal of the European Patent Office, No. 12/82